Europäisches Patentamt

European Patent Office

Office européen des brevets

(19)

(11) Publication number: **0 454 256 B1**

(12) **EUROPEAN PATENT SPECIFICATION**

(45) Date of publication of patent specification: **06.12.95**  (51) Int. Cl.6: **C07C 1/04**

(21) Application number: **91200963.6**

(22) Date of filing: **22.04.91**

(54) Process for the preparation of an olefins-containing mixture of hydrocarbons.

(30) Priority: **26.04.90 GB 9009392**

(43) Date of publication of application:
**30.10.91 Bulletin 91/44**

(45) Publication of the grant of the patent:
**06.12.95 Bulletin 95/49**

(84) Designated Contracting States:
**DE FR GB IT NL**

(56) References cited:
**EP-A- 0 037 213**
**GB-A- 2 073 237**
**US-A- 4 159 995**
**US-A- 4 304 871**

**CHEMICAL ABSTRACTS, vol. 107, 1987, Columbus, Ohio, US; abstract no. 118027v,**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Biswas, Jaydeep**
**Carel von Bylandtlaan 30**
**NL-2596 HR The Hague (NL)**
Inventor: **Tijm, Petrus Jacobus Adrianus**
**Badhuisweg 3**
**NL-1031 CM Amsterdam (NL)**
Inventor: **Eilers, Jacobus**
**Badhuisweg 3**
**NL-1031 CM Amsterdam (NL)**
Inventor: **Wielers, Antonius Franziskus Heinrich**
**Badhuisweg 3**
**NL-1031 CM Amsterdam (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 454 256 B1

**Description**

The present invention relates to a process for the preparation of an olefins-containing mixture of hydrocarbons.

There is considerable interest in the production of olefins, especially ethylene and propylene, as their reactivity renders them suitable for conversion to further products, in contrast to the low value lower paraffins.

It is known to convert hydrocarbonaceous feedstocks, such as light distillates, to products rich in lower olefins, especially ethylene and propylene, by high temperature steam cracking. The typical product slate obtained in such steam cracking processes is not entirely suited to the needs of the chemical industry in that it represents a comparatively low overall conversion to lower olefins, with a relatively high methane production level and a high ratio of ethylene to propylene.

From US-A-4159995 or Applied Catalysis vol. 32 No. 1-2 (1987) pp. 191-201 (Varma et al) is moreover taught to obtain olefins in low yield in a two stage process comprising contacting a synthesis gas (CO and $H_2$) with a Fischer Tropsch catalyst and contacting the product thereof with a catalyst comprising ZSM-5 at a temperature of 280 °C-343 °C and low space velocity in the range of 0.2-30 kg/kg.hr or at a temperature of 250 °C and increased time on stream respectively.

It has now been found that a comparatively high yield of olefins, with a relatively low methane yield, can be obtained from a very attractive two stage process wherein a gas mixture comprising carbon monoxide and hydrogen is used as starting material.

The present invention therefore relates to a process for the preparation of an olefins-containing mixture of hydrocarbons, which process comprises converting a gas mixture comprising carbon monoxide and hydrogen into a mixture of hydrocarbons by contacting it at elevated temperature and pressure with a catalyst in a first step, and contacting at least a fraction of the mixture of hydrocarbons obtained with a zeolitic catalyst comprising a zeolite with a pore diameter of 0.3 to 0.7 nm at a temperature above 480 °C during less than 10 seconds in a second step.

The catalytic reaction of the gas mixture of carbon monoxide and hydrogen at elevated temperature and pressure with a suitable catalyst is known in literature as the Fischer-Tropsch hydrocarbon synthesis process.

Catalysts often used for this purpose comprise one or more metals from group VIII of the Periodic Table, especially from the iron group, supported on a carrier, optionally in combination with one or more metal oxides and/or other metals as promoters. The metal oxide promoters are usually chosen from groups IIa, IIIb, IVb, and/or Vb of the Periodic Table as well as from the lanthanides and/or actinides. Very suitably magnesium, calcium, strontium, barium, scandium, yttrium, lanthanum, cerium, titanium, zirconium, hafnium, thorium, uranium, vanadium and chromium may be used. The metal promoter may be selected from the groups VIIb and/or VIII of the Periodic Table. Very suitably rhenium and group VIII noble metals (especially ruthenium, platinum and palladium) may be used. The amount of promoter is suitably between 0.1 and 150 pbw per 100 pbw carrier material.

Preferably, such a catalyst comprises 3-80 parts by weight of cobalt, especially 15-50 parts by weight of cobalt, and 0.1-100 parts by weight of at least one other metal selected from the group consisting of zirconium, titanium, chromium, rhenium and ruthenium, preferably 5-40 parts by weight of zirconium per 100 parts by weight carrier material. As carriers materials various refractory oxides may be used. Suitably, the carrier material comprises silica, alumina, silica-alumina, titania, zirconia, magnesia, ceria, gallia and mixtures thereof. Preferably silica is used. Preferably, the catalyst to be used in the first step comprises cobalt and zirconia as other metal and silica as carrier. Suitable techniques for the preparation of these catalysts comprise precipitation, impregnation, kneading and melting. Preferably, the catalyst has been prepared by kneading and/or impregnation. For further information on the preparation of the catalyst to be used in the first step reference is made to EP-A-127,220.

Suitably, the gas mixture comprising carbon monoxide and hydrogen can be prepared by the gasification of heavy carbonaceous materials such as coal and residual mineral oil fractions. It is preferred to start from a gas mixture which has been obtained by the steam reforming or partial oxidation of light hydrocarbons, in particular natural gas.

In the process according to the present invention the conversion of the gas mixture comprising carbon monoxide and hydrogen is preferably carried out at a temperature of 125-350 °C and a pressure of 5-100 bar and in particular at a temperature of 175-275 °C and a pressure of 10-75 bar.

At least a fraction of the mixture of hydrocarbons obtained in the first step is contacted with the zeolitic catalyst in the second step. Preferably, the fraction of the mixture of hydrocarbons to be contacted with the zeolitic catalyst in the second step consists substantially of paraffins, more preferably linear paraffins.

2

Advantageously, said fraction has an end boiling point ranging from 60 to 540 °C. In another suitable embodiment of the present invention the whole mixture of hydrocarbons obtained in the first step is contacted with the zeolitic catalyst in the second step. At least a fraction of the mixture of hydrocarbons obtained in the first step can be subjected to a catalytic hydrotreatment prior to contact with the zeolitic catalyst in the second step.

The catalytic hydrotreatment can be carried out by contacting the fraction to be treated at elevated temperature and pressure and in the presence of hydrogen with a catalyst containing one or more metals with hydrogenation activity supported on a carrier. Examples of suitable catalysts are catalysts containing nickel and/or cobalt and in addition molybdenum and/or tungsten supported on a carrier such as alumina or silica-alumina. In the catalytic hydrotreatment it is preferred to use a catalyst containing one or more noble metals from group VIII of the Periodic Table supported on a carrier. The quantity of the noble metal present in the catalyst may vary within wide limits, but is often between 0.05 and 5 %w. The noble metals from group VIII which may be present are platinum, palladium, ruthenium, iridium, osmium or mixtures thereof, platinum being preferred. The quantity of the group VIII metal in the catalyst is preferably 0.1 to 2 %w, and in particular 0.1 to 1 %w. Examples of suitable carriers are silica, alumina, magnesia, zirconia, zeolites and mixtures thereof, preferably a mixture of silica and alumina. Suitable conditions for carrying out the catalytic hydrotreatment are a temperature of 175 to 400 °C, a hydrogen partial pressure of 10 to 250 bar, a space velocity of 0.1 to 5 kg.$l^{-1}$.$h^{-1}$ and a hydrogen/oil ratio of 100 to 5000 Nl.$kg^{-1}$. The catalytic hydrotreatment is preferably carried out under the following conditions: a temperature of 250 to 350 °C, a hydrogen partial pressure between 25 and 100 bar, a space velocity of 0.25 to 2 kg.$l^{-1}$.$h^{-1}$ and a hydrogen/oil ratio of 250 to 2000 Nl.$kg^{-1}$.

The mixture of hydrocarbons obtained in the first step is contacted with the zeolitic catalyst for less than 10 seconds. Suitably, the minimum contact time is 0.1 second. Very good results are obtainable with a process in which the mixture of hydrocarbons is contacted with the zeolitic catalyst during 0.2 to 6 seconds.

The temperature during the reaction in the second step is relatively high. It is this combination of high temperature and short contact time which allows a high conversion to olefins. A preferred temperature range is 480 to 900 °C, more preferably 550 to 800 °C.

The zeolitic catalyst to be used in the second step may comprise one or more zeolites with a pore diameter of from 0.3 to 0.7 nm, preferably from 0.5 to 0.7 nm. The catalyst suitably further comprises a refractory oxide that serves as binder material. Suitable refractory oxides include alumina, silica, silica-alumina, magnesia, titania, zirconia and mixtures thereof. Alumina is especially preferred. The weight ratio of refractory oxide and zeolite suitably ranges from 10:90 to 90:10, preferably from 50:50 to 85:15. The zeolitic catalyst may comprise up to about 40% by weight of further zeolites with a pore diameter above 0.7 nm. Suitable examples of such zeolites include the faujasite-type zeolites, zeolite beta, zeolite omega and in particular zeolite X and Y. Preferably, the zeolitic catalyst consists substantially of a zeolite with a pore diameter of from 0.3 to 0.7 nm.

The term zeolite in this specification is not to be regarded as comprising only crystalline aluminium silicates. The term also includes crystalline silica (silicalite), silicoaluminophosphates (SAPO), chromosilicates, gallium silicates, iron silicates, aluminium phosphates (ALPO), titanium aluminosilicates (TAPO) and iron aluminosilicates.

Examples of zeolites that may be used in the second step of the process of the present invention and that have a pore diameter of 0.3 to 0.7 nm, include SAPO-4 and SAPO-11, which are described in US-A-4,440,871, ALPO-11, described in US-A-4,310,440, TAPO-11, described in US-A-4,500,651, TASO-45, described in EP-A-229,295, boron silicates, described in e.g. US-A-4,254,297, aluminium silicates like erionite, ferrierite, theta and the ZSM-type zeolites such as ZSM-5, ZSM-11, ZSM-12, ZSM-35, ZSM-23, and ZSM-38. Preferably, the zeolite is selected from the group consisting of crystalline metal silicates having a ZSM-5 structure, ferrierite, erionite and mixtures thereof. Suitable examples of crystalline metal silicates with ZSM-5 structure are aluminium, gallium, iron, scandium, rhodium and/or scandium silicates as described in e.g. GB-B-2,110,559.

During the preparation of the zeolites to be used in the second step usually a significant amount of alkali metal oxide is present in the prepared zeolite. Preferably, the amount of alkali metal is removed by methods known in the art, such as ion-exchange, optionally followed by calcination, to yield the zeolite in its hydrogen form. Preferably, the zeolite used in the second step is substantially in its hydrogen form. Olefin production is facilitated by the absence of hydrogen or a hydrogen donor. Hence, the present process is advantageously carried out in the absence of added hydrogen and/or steam. It is, of course, possible that during the reaction some small molecules, such as hydrogen molecules are formed.

The pressure in the second step of the process according to the present invention can be varied within wide ranges. It is, however, preferred that the pressure is such that at the prevailing temperature the mixture of hydrocarbons obtained in the first step is substantially in its gaseous phase or brought thereinto by contact with the catalyst. This can be advantageous since no expensive compressors and high-pressure vessels and other equipment are necessary. A suitable pressure range is from 1 to 10 bar. Subatmospheric pressures are possible, but not preferred. It can be economically advantageous to operate at atmospheric pressure. Other gaseous materials may be present during the conversion of the mixture of hydrocarbons such as steam and/or nitrogen.

The second step of the process according to the present invention can suitably be carried out in either a fixed bed or a moving bed of catalyst. Preferably, the second step of the process according to the present invention is carried out in a moving bed. The bed of catalyst may move upwards or downwards. When the bed moves upwards a process somewhat similar to a fluidized catalytic cracking process is obtained.

In the second step some coke forms on the catalyst. Therefore it is advantageous to regenerate the catalyst. Preferably, the catalyst is regenerated by subjecting it, after having been contacted with the mixture of hydrocarbons, to a treatment with an oxidizing gas, such as air. A continuous regeneration, similar to the regeneration carried out in a fluidized catalytic cracking process, is especially preferred.

If the coke formation does not occur at too high a rate, it would be possible to arrange for a second process step in which the residence time of the catalyst particles in a reaction zone is longer than the residence time of the mixture of hydrocarbons in the reaction zone. Of course the contact time between the mixture of hydrocarbons and catalyst should be less than 10 seconds. The contact time generally corresponds with the residence time of the mixture of hydrocarbons to be converted. Suitably, the residence time of the catalyst is from 1 to 10 times the residence time of the mixture of hydrocarbons. One of the advantages of the present process is that in the second step only small amounts of coke are formed, enabling the use of a fixed bed of catalyst in the second step of the present process. Suitably, methanol is co-processed in the second step to obtain even more advanced lower olefins yields.

The weight ratio of the catalyst used in the second step relative to the fraction of the mixture of hydrocarbons to be converted (catalyst/oil ratio, g/g) may vary widely, for example up to 150 kg of catalyst per kg of the fraction of the mixture of hydrocarbons or even more. Preferably, the weight ratio of catalyst used in the second step relative to the fraction of the mixture of hydrocarbons is from 20 to 100:1.

Advantageously, the unconverted liquid fraction of the mixture of hydrocarbons is subjected to the second step of the present process again. Suitably, the unconverted liquid fraction of the mixture of hydrocarbons is recycled to the reaction zone wherein the second step of the present process is carried out.

The invention will now be illustrated by way of the following Example.

EXAMPLE

Use was made of the following catalysts:

Catalyst 1

Co/Zr/SiO$_2$ catalyst containing 25 parts by weight of cobalt and 1.8 parts by weight of zirconium per 100 parts by weight of silica, which was prepared by impregnating a silica carrier with an aqueous solution containing a cobalt and a zirconium salt, followed by drying the composition, calcining at 500 °C and reducing at 280 °C.

Catalyst 2

Catalyst comprising ZSM-5 in an alumina matrix (weight ratio ZSM-5/alumina 1:3).

Preparations of olefins-containing mixtures of hydrocarbons

An H$_2$/CO mixture having a H$_2$/CO molar ratio of 1.1 and obtained by subjecting a natural gas to a partial oxidation process was contacted in a first step at a temperature of 220 °C and a pressure of 30 bar with catalyst 1.

Subsequently the total reaction product of the first step was contacted in a second step with catalyst 2 in a moving bed. The process conditions of step 2 and the results obtained are shown in Table 1 below.

4

## Table 1

Process conditions step 2:

| | | | |
|---|---|---|---|
| Reactor temperature, °C | 580 | 670 | 700 |
| Catalyst/oil ratio, g/g | 86 | 69 | 65 |
| Contact time, s | 1.4 | 1.4 | 1.4 |
| Pressure, bar | 2 | 2 | 2 |

Product, % wt on natural gas

| | | | |
|---|---|---|---|
| $C_1$ | 0.3 | 3.3 | 7.4 |
| $C_2^-$ | 0.6 | 2.0 | 3.1 |
| $C_2^=$ | 5.8 | 11.9 | 19.2 |
| $C_3^-$ | 4.3 | 2.6 | 1.6 |
| $C_3^=$ | 23.7 | 25.9 | 27.3 |
| $C_4^-$ | 4.3 | 2.5 | 1.3 |
| $C_4^=$ | 18.5 | 17.8 | 15.6 |
| $C_5$-221 °C | 34.6 | 26.1 | 18.2 |
| 221-370 °C | 4.7 | 3.8 | 2.8 |
| Coke | 3.2 | 4.1 | 3.5 |

From the above results it will be clear that the present process is very attractive for obtaining a high proportion of olefinic products from a gas mixture comprising carbon monoxide and hydrogen, whereby only little coke is made in the second step of the process.

## Claims

1. Process for the preparation of an olefins-containing mixture of hydrocarbons, which process comprises converting a gas mixture comprising carbon monoxide and hydrogen into a mixture of hydrocarbons by contacting it at elevated temperature and pressure with a catalyst in a first step, and contacting at least a fraction of the mixture of hydrocarbons obtained with a zeolitic catalyst comprising a zeolite with a pore diameter of 0.3 to 0.7 nm at a temperature above 480 °C during less than 10 seconds in a second step.

2. Process according to claim 1, wherein a gas mixture is used which has been obtained, starting from light hydrocarbons, by steam reforming or partial oxidation.

3. Process according to claim 2, wherein the gas mixture which is used has been obtained starting from natural gas.

4. Process according to any one of claims 1-3, wherein the catalyst used in the first step comprises one or more metals from group VIII supported on a carrier, optionally in combination with one or more metal oxides from groups IIa, IIIb, IVb and/or Vb, and/or other metals from groups VIIb and/or VIII as promoters.

5. Process according to any one of claims 1-4, wherein the catalyst used in the first step comprises 3-80 pbw of Co and 0.1-100 pbw of at least one other metal chosen from the group formed by Zr, Ti, Re, Ru and Cr per 100 pbw of silica, alumina, silica-alumina or titania carrier.

**6.** Process according to any one of claims 1-5, wherein the catalyst used in the first step has been prepared by kneading and/or impregnation.

**7.** Process according to claim 5 or 6, wherein the catalyst used in the first step comprises Co and Zr and silica as carrier.

**8.** Process according to any one of claims 1-7, wherein the fraction of the mixture of hydrocarbons consists substantially of paraffins.

**9.** Process according to any one of claims 1-8, wherein the fraction has an end boiling point in the range of 60-540 °C.

**10.** Process according to any one of claims 1-9, wherein the conversion of the $H_2/CO$ mixture in the first step is carried out at a temperature of 125-350 °C and a pressure of 5-100 bar.

**11.** Process according to any one of claims 1-10, wherein the fraction of the mixture of hydrocarbons is contacted in the second step with the zeolitic catalyst during 0.2 to 6 seconds.

**12.** Process according to any one of claims 1-11, wherein the temperature in the second step is from 480 to 900 °C.

**13.** Process according to claim 12, wherein the temperature in the second step is from 550 to 800 °C.

**14.** Process according to any one of claims 1-13, wherein the zeolite used in the second step has a pore diameter of 0.5 to 0.7 nm.

**15.** Process according to any one of claims 1-14, wherein the zeolite used in the second step is selected from crystalline metal silicates having a ZSM-5 structure, ferrierite, erionite and mixtures thereof.

**16.** Process according to any one of claims 1-15, wherein the zeolite used in the second step is substantially in its hydrogen form.

**17.** Process according to any one of claims 1-16, wherein the pressure in the second step is from 1 to 10 bar.

**18.** Process according to any one of claims 1-17, wherein the weight ratio of the catalyst used in the second step relative to the fraction of the mixture of hydrocarbons is from 20 to 100:1.

**19.** Process according to any one of claims 1-18, wherein the second step is carried out in a moving bed of catalyst.

**20.** Process according to any one of claims 1-19, wherein the unconverted liquid fraction of the mixture of hydrocarbons is subjected to the second step of the process again.

**Patentansprüche**

**1.** Verfahren zur Herstellung eines olefinhaltigen Kohlenwasserstoffgemisches, wobei man ein Kohlenmonoxid und Wasserstoff enthaltendes Gasgemisch in ein Kohlenwasserstoffgemisch überführt, indem man es in einem ersten Schritt bei erhöhter Temperatur und erhöhtem Druck mit einem Katalysator in Kontakt bringt, und zumindest einen Teil des erhaltenen Kohlenwasserstoffgemisches in einem zweiten Schritt bei einer Temperatur von mehr als 480 °C für weniger als 10 Sekunden mit einem zeolithhaltigen Katalysator in Kontakt bringt, welcher einen Zeolith mit einem Porendurchmesser von 0,3 bis 0,7 nm enthält.

**2.** Verfahren nach Anspruch 1, wobei man ein Gasgemisch verwendet, das man, von leichten Kohlenwasserstoffen ausgehend, durch Dampfreformieren oder Teiloxidation erhalten hat.

3. Verfahren nach Anspruch 2, wobei man das verwendete Gasgemisch ausgehend von Erdgas erhalten hat.

4. Verfahren nach einem der Ansprüche 1-3, wobei der in dem ersten Schritt eingesetzte Katalysator ein oder mehrere Metalle der Gruppe VIII, gegebenenfalls in Kombination mit einem oder mehreren Metalloxiden der Gruppen IIa, IIIb, IVb und/oder Vb und/oder weiteren Metallen der Gruppen VIIb und/oder VIII als Promotoren, auf einem Träger enthält.

5. Verfahren nach einem der Ansprüche 1-4, wobei der in dem ersten Schritt eingesetzte Katalysator 3-80 Gewichtsteile Co und 0,1-100 Gewichtsteile mindestens eines weiteren Metalles, welches man aus der von Zr, Ti, Re, Ru und Cr gebildeten Gruppe auswählt, je 100 Gewichtsteile Siliciumdioxid-, Aluminiumoxid-, Siliciumdioxid/Aluminiumoxid- oder Titandioxid-Träger enthält.

6. Verfahren nach einem der Ansprüche 1-5, wobei der in dem ersten Schritt eingesetzte Katalysator durch Kneten und/oder Imprägnieren hergestellt wird.

7. Verfahren nach Anspruch 5 oder 6, wobei der in dem ersten Schritt eingesetzte Katalysator Co und Zr und Siliciumdioxid als Träger enthält.

8. Verfahren nach einem der Ansprüche 1-7, wobei der besagte Teil des Kohlenwasserstoffgemisches weitgehend aus Paraffinen besteht.

9. Verfahren nach einem der Ansprüche 1-8, wobei der besagte Teil einen Endsiedepunkt im Bereich von 60-540 °C aufweist.

10. Verfahren nach einem der Ansprüche 1-9, wobei man die Umsetzung des $H_2$/CO-Gemisches im ersten Schritt bei einer Temperatur von 125-350 °C und einem Druck von 5-100 bar durchführt.

11. Verfahren nach einem der Ansprüche 1-10, wobei man den besagten Teil des Kohlenwasserstoffgemisches im zweiten Schritt für 0,2 bis 6 Sekunden mit dem zeolithhaltigen Katalysator in Kontakt bringt.

12. Verfahren nach einem der Ansprüche 1-11, wobei die Temperatur im zweiten Schritt 480 bis 900 °C beträgt.

13. Verfahren nach Anspruch 12, wobei die Temperatur im zweiten Schritt 550 bis 800 °C beträgt.

14. Verfahren nach einem der Ansprüche 1-13, wobei der im zweiten Schritt eingesetzte Zeolith einen Porendurchmesser von 0,5 bis 0,7 nm aufweist.

15. Verfahren nach einem der Ansprüche 1-14, wobei man den im zweiten Schritt eingesetzten Zeolith aus kristallinen Metallsilikaten mit ZSM-5-Struktur, Ferrierit, Erionit und Mischungen daraus auswählt.

16. Verfahren nach einem der Ansprüche 1-15, wobei der im zweiten Schritt eingesetzte Zeolith weitgehend in seiner Wasserstofform vorliegt.

17. Verfahren nach einem der Ansprüche 1-16, wobei der Druck im zweiten Schritt 1 bis 10 bar beträgt.

18. Verfahren nach einem der Ansprüche 1-17, wobei das Gewichtsverhältnis des im zweiten Schritt eingesetzten Katalysators zum besagten Teil des Kohlenwasserstoffgemisches 20 bis 100:1 beträgt.

19. Verfahren nach einem der Ansprüche 1-18, wobei man den zweiten Schritt in einem Katalysator-Fließbett durchführt.

20. Verfahren nach einem der Ansprüche 1-19, wobei man die nichtumgesetzte flüssige Fraktion des Kohlenwasserstoffgemisches nochmals dem zweiten Schritt des Verfahrens unterwirft.

**Revendications**

1. Procédé de préparation d'un mélange d'hydrocarbures contenant des oléfines, ce procédé comprenant la conversion d'un mélange gazeux comprenant du monoxyde de carbone et de l'hydrogène en un mélange d'hydrocarbures, par mise en contact de celui-ci, à une température et sous une pression élevées, avec un catalyseur dans une première étape, et mise en contact d'au moins une fraction du mélange d'hydrocarbures obtenu avec un catalyseur zéolitique comprenant une zéolite ayant un diamètre de pores de 0,3 à 0,7 nanomètre à une température supérieure à 480°C, pendant moins de 10 secondes, dans une seconde étape.

2. Procédé selon la revendication 1, dans lequel on utilise un mélange gazeux ayant été obtenu à partir d'hydrocarbures légers par vaporeformage ou par oxydation partielle.

3. Procédé selon la revendication 2, dans lequel le mélange gazeux utilisé a été obtenu à partir de gaz naturel.

4. Procédé selon l'une quelconque des revendications 1-3, dans lequel le catalyseur utilisé dans la première étape comprend un ou plusieurs métaux du groupe VIII supporté sur un support, éventuellement en combinaison avec un ou plusieurs oxydes métalliques des groupes IIA, IIIB, IVB et/ou VB, et/ou d'autres métaux des groupes VIIB et/ou VIII, comme promoteurs.

5. Procédé selon l'une quelconque des revendications 1-4, dans lequel le catalyseur utilisé dans la première étape comprend 3-80 parties en poids de Co et 0,1-100 parties en poids d'au moins un autre métal choisi dans le groupe formé par Zr, Ti, Re, Ru et Cr, pour 100 parties en poids de support silice, alumine, silice-alumine ou oxyde de titane.

6. Procédé selon l'une quelconque des revendications 1-5, dans lequel le catalyseur utilisé dans la première étape a été préparé par pétrissage et/ou imprégnation.

7. Procédé selon la revendication 5 ou 6, dans lequel le catalyseur utilisé dans la première étape comprend Co et Zr, et de la silice comme support.

8. Procédé selon l'une quelconque des revendications 1-7, dans lequel la fraction du mélange d'hydrocarbures est essentiellement constituée de paraffines.

9. Procédé selon l'une quelconque des revendications 1-8, dans lequel la fraction possède un point d'ébullition final dans la gamme de 60-540°C.

10. Procédé selon l'une quelconque des revendications 1-9, dans lequel la conversion du mélange $H_2$/CO dans la première étape est réalisée à une température de 125-350°C et sous une pression de 5-100 bars.

11. Procédé selon l'une quelconque des revendications 1-10, dans lequel la fraction du mélange d'hydrocarbures est mise en contact, dans la seconde étape, avec le catalyseur zéolitique pendant 0,2 à 6 secondes.

12. Procédé selon l'une quelconque des revendications 1-11, dans lequel la température, dans la seconde étape, est de 480 à 900°C.

13. Procédé selon la revendication 12, dans lequel la température, dans la seconde étape, est de 550 à 800°C.

14. Procédé selon l'une quelconque des revendications 1-13, dans lequel la zéolite utilisée dans la seconde étape possède un diamètre de pores de 0,5 à 0,7 nm.

15. Procédé selon l'une quelconque des revendications 1-14, dans lequel la zéolite utilisée dans la seconde étape est choisie parmi les silicates métalliques cristallins ayant une structure ZSM-5, la ferriérite, l'érionite, et leurs mélanges.

**16.** Procédé selon l'une quelconque des revendications 1-15, dans lequel la zéolite utilisée dans la seconde étape est essentiellement sous sa forme hydrogénée.

**17.** Procédé selon l'une quelconque des revendications 1-16, dans lequel la pression, dans la seconde étape, est de 1 à 10 bars.

**18.** Procédé selon l'une quelconque des revendications 1-17, dans lequel le rapport pondéral du catalyseur utilisé dans la seconde étape par rapport à la fraction du mélange d'hydrocarbures est de 20 à 100:1.

**19.** Procédé selon l'une quelconque des revendications 1-18, dans lequel la seconde étape est réalisée dans un lit mobile de catalyseur.

**20.** Procédé selon l'une quelconque des revendications 1-19, dans lequel la fraction liquide non convertie du mélange d'hydrocarbures est soumise une nouvelle fois à la seconde étape du procédé.